# EUROPEAN PATENT SPECIFICATION

(11) **EP 3 328 313 B1**
(45) Date of publication and mention of the grant of the patent: **12.06.2019**
(21) Application number: 16757080.3
(22) Date of filing: 22.07.2016
(51) Int. Cl.: A61C 8/00, A61L 27/00, A61C 8/02, A61L 27/36, A61K 35/32

(54) **APPARATUS FOR PROCESSING TEETH TO BONE MATERIAL**
VORRICHTUNG ZUR VERARBEITUNG VON ZÄHNEN ZU KNOCHENMATERIAL
APPAREIL DE TRAITEMENT DE DENTS EN MATÉRIAU OSSEUX

(30) Priority: 28.07.2015 IT UB20152560
(43) Date of publication of application: 06.06.2018
(73) Proprietor: TT Tooth Transformer S.r.l., 20146 Milan (IT)
(72) Inventor: MINETTI, Elio, I - 38088 SPIAZZO (Trento) (IT); ARU, Claudio, I - 10143 Torino (IT)
(74) Representative: Lunati & Mazzoni S.r.L.
(86) International application number: PCT/IB2016/054386
(87) International publication number: WO 2017/017577

(56) References cited:
- EP-A2- 1 121 942
- WO-A1-2014/111925
- WO-A2-03/020107
- KR-A- 20140 018 455
- KR-B1- 101 299 395
- DATABASE WPI Week 201263 Thomson Scientific, London, GB; AN 2012-L96104 XP002751828, & WO 2012/121448 A1 (DASTECH CO LTD) 13 September 2012 (2012-09-13)

## Description

The object of the present invention concerns apparatus for processing teeth in bone material of the type specified in the preamble of the first claim.

Procedures and methods for processing a tooth or portion of tooth in bone material suitable for constituting the base to support a new tooth or for other purposes are currently known.

Similar apparatus are described in patent applications: EP 1121942 A2, WO 2014/111925 A1; WO 03/020107 A2, KR 2014 0018455 A, KR 101 299 395 B1, WO 2012/121448 A1.

In particular, the tooth is extracted, washed, broken down and treated with different liquids.

The treatment is carried out, in particular, with mixtures of chloroform, methanol, hydrochloric acid and hydrogen peroxide, for several hours or even days.

The processed tooth is thus a bone powder with the same DNA as the bone of the patient whose tooth was extracted.

The thus formed bone can be used on the same patient as the base for an implant and for the formation and integration of said implant with the bone, for example of the jaw bone, where an artificial tooth is to be fitted.

Said process has been known for about twenty years and is described, for example in the Korean patent applications KR-A-20030068957 and KR-A-19980008980, and also in the scientific article entitled "Development of a novel bone grafting material using autogenous teeth" and published in the journal Oral Surg Oral Med Oral Pathol Oral Radiol Endod 2010;109:496-503.

Said procedure is extremely advantageous in that it allows greater integration of a prosthesis, in particular of a dental type, on a patient's bones, as well as improved patient recovery.

Apparatus has been produced to realise said process.

However, said apparatus presents important inconveniences.

In fact, said apparatus is extremely complex and costly and furthermore it does not guarantee patient sterility and it does not protect the patient against the risk of cross-infection.

Due to its complexity, it is subject to malfunctioning.

Lastly, said apparatus does not allow a precise and safe dosage and composition of the treatment liquids.

In this situation, the technical task at the base of the present invention is to create an apparatus for processing teeth in bone material, which can substantially remedy the aforesaid inconveniences.

In the sphere of said technical task, it is an important scope of the invention to obtain apparatus for processing teeth in bone material, which is simple, cheap and robust. Another important purpose of the invention is to create apparatus for processing teeth in bone material that allows a precise and unequivocal supply of treatment liquids.

The technical task and specified purposes are reached in accordance with the annexed claim 1.

Preferred embodiments are highlighted in the dependent claims.

The characteristics and advantages of the invention are subsequently clarified by the detailed description of preferred embodiments of the invention, with reference to the accompanying drawings, wherein:

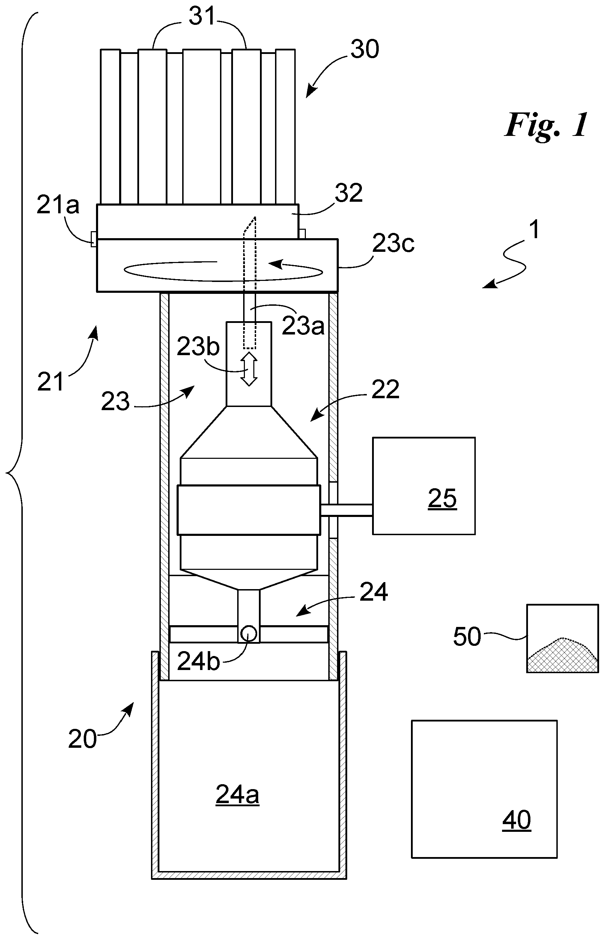

**Drawing 1** shows a diagram of the apparatus for processing teeth in bone material according to the invention.

With reference to the Drawing, the apparatus for processing teeth in bone material according to the invention is globally indicated with number **1.**

It acts in accordance with previously described processes and reactions, in a known manner and is suitable for processing a tooth, preferably a human tooth, in bone material to support a new tooth or for other purposes.

In short, the apparatus 1 comprises a reactor device **20** to realise the chemical reactions, in particular, with liquids, a cartridge **30** for containing the washing and processing liquids, a fragmentation device **40** for teeth, suitable for fragmenting the teeth, a container for fragments **50** of teeth suitable for being placed in the reactor 20.

In detail, the cartridge 30 comprises a plurality of containers **31,** for the processing and washing liquids, separated liquid-tight from each other.

The cartridge 30 is preferably cylindrical and the containers 31 are angular sectors of the same cartridge. Preferably, 8 containers are present, at least three of which for processing, in detail, acid solutions, buffer solutions and alcohol, and at least four for washing. The containers 31 are suitable for containing, and preferably, each one contains an adequate and necessary quantity for one single operation. The cartridge 30 furthermore comprises engaging means with the reactor device 20. The cartridge 30 furthermore comprises a support element **32,** as a base or similar, and means for indicating the position (not illustrated) suitable for allowing the device 20 to read the position of the cartridge 30 and, in particular of the tanks 31. Finally, the cartridge 30 is preferably of a disposable type.

The reactor device 20 is suitable for treating the teeth by immersion in liquids. Furthermore, in short, it comprises connection means **21** with the cartridge 30, suitable for putting the cartridge 30 in mechanical connection with the reactor device 20, a reaction chamber **22,** suitable for containing the teeth during processing, and the processing and washing liquids, commandable transfer means **23** of the liquids from the containers 31 to the reaction chamber 22 and ejection means **24** of the liquids in the reaction chamber 22.

In greater detail, the connection means 21 comprise a mechanical support **21a,** of a known type, such as a container or the like, for the cartridge 30, which is preferably suitable for blocking the cartridge 30 in a vertical direction.

The reaction chamber 22 is suitable for containing the teeth and liquids during the reactions and washes. It is preferably made of metal, more preferably in stainless steel, and is suitable for being sterilised in autoclave or disposable. The reaction chamber 22 preferably has a cylindrical shape and a central portion with a greater diameter, where said reactions take place. Furthermore, it may comprise openings for loading the teeth or for inspections.

Level with the reaction chamber 22, heating and vibrating means **25** are also present for the reaction chamber 22 and the liquids contained, preferably of an electrical type and preferably elastically connectable and separable from the reaction chamber 22.

The commandable transfer means 23 are suitable for placing one single container 31 at a time in fluidic connection with the reaction chamber 22.

Furthermore, they are opportunely suitable for transferring the liquid from the containers 31 to the reaction chamber 22 by gravity. Furthermore, advantageously, the transfer means 23 are suitable for transferring all of the liquid contained in a said container 31 continuously and do not comprise check valves for the flow of fluid. All of the contents of the containers 31 can and must be poured into the reaction chamber 22 in one single operation, since the liquid is already correctly dosed and made up in the cartridge. The reactor device 20 therefore has a simple and compact structure.

Preferably, the transfer means 23 consist of a hollow needle **23a** and first handling means **23b** of said needle 23a. The needle 23a, is therefore suitable for perforating one of the containers set vertically above the same needle 23a.

The transfer means 23 furthermore comprise second handling means **23c** of the cartridge 30, suitable for positioning a specific tank above the needle 23a and consequently allowing the connection between the same hollow needle 23a and a selected tank 31. The second handling means 21c preferably comprise a rotary engine, opportunely electric, more preferably a stepper motor. Lastly, the connection means 21 comprise means for reading (not illustrated) the position of the cartridge 31, interacting with the means for indicating the position of the cartridge 30 that are suitable for allowing the device 20 to read the position of the cartridge 30 and in particular of the tanks 31 in relation to the device 20 and in particular to the needle 23a.

The ejection means 24 preferably comprise a drainage tank **24a,** suitable for containing the liquids after the treatment that are preferably joined in a releasable manner to the rest of the device 20. The drainage tank 24a and the reaction chamber 22 are preferably jointly connected for the passage of fluid and separated by a discharge valve **24b,** which can be activated electrically.

Preferably, the transfer of fluids from the reaction chamber 22 to the drainage tank 24a takes place by gravity.

The fragmentation device 40 of the tooth is suitable for fragmenting said tooth, or a portion of tooth, into particles of between 0.3 mm and 0.8 mm in diameter. It comprises a closed container and an electric mill, housed in said container.

Finally, the container for fragments 50 of teeth is suitable for being placed inside the reaction chamber 22, which is suitable for keeping said fragments of teeth inside it and permeable to the liquids during the reactions, so the fragments of tooth come into contact with the washing and reaction liquids.

The working of the apparatus 1 for processing teeth in bone material, previously described in structural terms, is as follows.

A tooth is extracted from the patient.

The same tooth is put into the container of the fragmentation device 40 where it is broken down by the electric mill into particles of between 0.3 mm and 0.8 mm in diameter.

The fragments are placed in the container 50, which is, in turn, in the reaction chamber 22 of the device 20.

At the same time, a cartridge 30 is positioned level with the connection means 21. The reactor apparatus 20 is then started.

The means for reading the position of the cartridge 31, part of the device 20, read the position of the containers 31 of the cartridge 30, and the second handling means 23c of the cartridge 30, position a special tank above the needle 23a. The first handling means 23b move the hollow needle 23, which perforates the selected container 31.

The liquids contained in the selected container 31 fall into the reaction chamber 22 and come into contact with the container 50, triggering a special reaction or wash with the fragments of tooth.

When the treatment, which is carried out in a set period of time, is finished, the drainage valve 24b is opened with the special liquid and the liquid is transferred to the drainage tank 24a.

Then the cartridge 30 can be moved again, by the second handling means 23c, and a new container can be 30 perforated and its contents transferred to the reaction chamber 22 and subsequently to the drainage tank 24a.

Preferably, wash cycles are alternated with washing and reaction liquids, preferably carried out with HCl, H₂O₂ and alcohol, with times of a known type.

When said operations are finished, the container 50 is extracted and the fragments of teeth, processed into bone fragments, are extracted and utilised.

The cartridge 30, which is now empty, is thrown away and at least portions of the reaction device 20, as well as the container for fragments 50 and the fragmentation device 40 are sterilized in autoclave. Alternatively, the container for fragments 50 is of a disposable type.

The apparatus for processing teeth in bone material according to the invention achieves important advantages.

In fact, the apparatus 1 is very simple, cheap and robust.

Furthermore, the use of the pre-loaded disposable cartridge 30 allows precise dosing and correct composition of the washing and reaction liquids.

## Claims

1. Apparatus (1) for processing teeth to bone material comprising a reactor device (20), suitable to treat said teeth by immersion in liquids, said reactor device (20) comprising:
- connection means (21) to a cartridge (30), comprising a plurality of containers (31) for said processing and washing liquids, separated liquid-tight from each other,
- and being **characterised in that** it comprises:
- a reaction chamber (22) suitable to contain said teeth during said processing and said processing and washing liquids,
- commandable transfer means (23) of said liquids from said containers (31) to said reaction chamber (22) suitable to place in fluidic connection only one at a time of said containers (31) with said reaction chamber (22),
- ejection means (24) of said liquids from said reaction chamber (22).
- said transfer means (23) being configured to transfer by gravity said liquid from said containers (31) to said reaction chamber (22).

2. Apparatus (1) according to one or more of the previous claims, wherein said transfer means (23) are suitable to transfer all the liquid contained in a said container (31) continuously and do not comprise check valves of said fluid.

3. Apparatus (1) according to one or more of the previous claims, wherein said transfer means (23) comprise handling means (23c) of said cartridge (30), so as to place a selected container (31) in a specific position suitable to allow said arrangement in a fluidic through connection of said container (31) with said reaction chamber (22).

4. Apparatus (1) according to one or more of the previous claims, wherein said ejection means (24) comprise a drainage tank (24a).

5. Apparatus (1) according to the previous claim, wherein said ejection means (24) are suitable to transfer by gravity said liquid from said reaction chamber (22) to said drainage tank (24a).

6. Apparatus (1) according to one or more of the previous claims, wherein said reactor device (20) comprises heating and vibrating means (25) for said reaction chamber (22) and the liquids contained therein.

7. Apparatus (1) according to one or more of the previous claims, comprising said cartridge (30), wherein said cartridge (30) is a disposable cartridge.

8. Apparatus (1) according to one or more of the previous claims, comprising a fragmentation device (40) of a tooth, suitable to fragment a tooth, or a portion of tooth, into particles of between 0.3 mm and 0.8 mm in diameter.

9. Apparatus (1) according to one or more of the previous claims, comprising a container for fragments (50) of teeth, suitable to be placed inside said reaction chamber (22), suitable to keep said tooth fragments inside it during the reactions, and permeable to liquids.

## Patentansprüche

1. Vorrichtung (1) zur Verarbeitung von Zähnen zu Knochenmaterial, umfassend eine Reaktorvorrichtung (20) die geeignet ist, Zähne durch Eintauchen in Flüssigkeiten zu verarbeiten,
wobei die genannte Reaktorvorrichtung (20) Folgendes umfasst:
- Elemente zur Verbindung (21) mit einer Patrone (30), die eine Vielzahl von Behältern (31) für die genannten Verarbeitungs- und Waschflüssigkeiten umfasst, die voneinander flüssigkeitsdicht getrennt sind,
- und **dadurch gekennzeichnet, dass** sie Folgendes umfasst:
- eine Reaktionskammer (22), die geeignet ist, die genannten Zähne während der genannten Verarbeitung und die genannten Verarbeitungs- und Waschflüssigkeiten zu enthalten,
- Übertragungselemente (23) zur Steuerung der genannten Flüssigkeiten von den genannten Behältern (31) zu der genannten Reaktionskammer (22), die geeignet sind, jeweils nur einen der genannten Behälter (31) mit der genannten Reaktionskammer (22) für den Flüssigkeitsdurchsatz zu verbinden,
- Vorrichtungen zum Ausstoßen (24) der genannten Flüssigkeiten aus der genannten Reaktionskammer (22),
- wobei die genannten Übertragungselemente (23) dazu konfiguriert sind, durch Gravitation die genannte Flüssigkeit von den genannten Behältern (31) zu der genannten Reaktionskammer (22) zu leiten.

2. Vorrichtung (1) nach einem oder mehreren der vorangegangenen Ansprüche, bei der die genannten Übertragungselemente (23) geeignet sind, die gesamte in einem genannten Behälter (31) enthaltene Flüssigkeit kontinuierlich zu übertragen und keine Absperrventile des genannten Fluides umfassen.

3. Vorrichtung (1) nach einem oder mehreren der vorangegangenen Ansprüche, bei der die genannten Übertragungselemente (23) Elemente zum Bewegen (23c) der genannten Patrone (30) umfassen, um einen ausgewählten Behälter (31) in eine spezifische Position zu bringen, die geeignet ist, die genannte Anordnung zur Verbindung für den Flüssigkeitsdurchsatz des ausgewählten Behälters (31) mit der genannten Reaktionskammer (22) zu gestatten.

4. Reaktionskammer (1) nach einem oder mehreren der vorangegangenen Ansprüche, bei der die genannten Elemente zum Ausstoßen (24) einen Ablassbehälter (24a) umfassen.

5. Vorrichtung (1) nach dem vorangegangenen Vorgang, bei der die genannten Elemente zum Ausstoßen (24) geeignet sind, die genannte Flüssigkeit durch Gravitation von der genannten Reaktionskammer (22) an den genannten Ablassbehälter (24a) zu übertragen.

6. Vorrichtung (1) nach einem oder mehreren der vorangegangenen Ansprüche, bei der die genannte Reaktorvorrichtung (20) Heiz- und Rüttelvorrichtungen (25) für die genannte Reaktionskammer (22) und die darin enthaltenen Flüssigkeiten umfasst.

7. Vorrichtung (1) nach einem oder mehreren der vorangegangenen Ansprüche, umfassend die genannte Patrone (30), bei der es sich bei der genannten Patrone (30) um eine Einwegpatrone handelt.

8. Vorrichtung (1) nach einem oder mehreren der vorangegangenen Ansprüche, umfassend eine Vorrichtung zum (40) Zerkleinern eines Zahns, die in der Lage ist, einen Zahn oder einen Teil eines Zahns zu Partikeln mit einem Durchmesser zwischen 0,3 mm und 0,8 mm zu zerkleinern.

9. Vorrichtung (1) nach einem oder mehreren der vorangegangenen Ansprüche, umfassend einen Behälter für Zahnfragmente (50), der geeignet ist, im Inneren der genannten Reaktionskammer (22) positioniert zu werden und während der Reaktionen die genannten Zahnfragmente in seinem Inneren und für die Flüssigkeiten durchlässig zu enthalten.

## Revendications

1. Appareil (1) pour la transformation des dents en matériel osseux comprenant un dispositif réacteur (20) en mesure de traiter lesdits dents par immersion dans des liquides,
ledit dispositif réacteur (20) comprenant :
- des moyens de connexion (21) avec une cartouche (30) comprenant plusieurs conteneurs (31) pour les liquides de transformation et de lavage, séparés entre eux par étanchéité aux liquides,
- et **se caractérisant par** le fait de comprendre :
- une chambre de réaction (22) propre à contenir les dents pendant la transformation et les liquides de transformation et de lavage,
- les moyens de transfert (23) commandant les liquides à partir des conteneurs (31) vers ladite chambre de réaction (22) propre à mettre en connexion pour le passage de liquides un seul desdits connecteurs (31) à la fois avec la chambre de réaction (22).
- les moyens d'expulsion (24) desdits liquides de la chambre de réaction (22),
- lesdits moyens de transfert (23) sont configurés pour transférer ledit liquide par chute desdits conteneurs (31) vers ladite chambre de réaction (22).

2. Appareil (1) selon une ou plusieurs des revendications précédentes dans lequel les moyens de transfert (23) sont en position de transférer tout le liquide contenu dans un conteneur (31) en façon continue et ne contiennent pas de soupapes d'arrêt pour ledit fluide.

3. Appareil (1) selon une ou plusieurs des revendications précédentes dans lequel lesdits moyens de transfert (23) comprennent des moyens de déplacement (23c) de la cartouche (30) de sorte à disposer un conteneur (31) choisi dans une position spécifique propre à permettre la disposition en raccord pour le passage de fluide du conteneur (31) choisi à la chambre de réaction.

4. Appareil (1) selon une ou plusieurs des revendications précédentes dans lequel les moyens d'expulsion (24) comprennent un réservoir d'évacuation (24a).

5. Appareil (1) selon une ou plusieurs des revendications précédentes dans lequel lesdits moyens d'expulsion (24) sont propres à transférer le liquide par chute de la chambre de réaction (22) au réservoir d'évacuation (24a) par chute.

6. Appareil (1) selon une ou plusieurs des revendications précédentes dans lequel ledit dispositif réacteur (20) comprend des moyens de réchauffement et vibrants (25) pour la chambre de réaction (22) et les liquides contenus.

7. Appareil (1) selon une ou plusieurs des revendications précédentes et comprenant ladite cartouche (30) et dans lequel ladite cartouche (30) est une cartouche jetable.

8. Appareil (1) selon une ou plusieurs des revendications précédentes et comprenant un dispositif de fragmentation (40) d'une dent propre à fragmenter une dent ou une portion de dent en particules d'un diamètre compris entre 0,3 mm et 0,8 mm.

9. Appareil (1) selon une ou plusieurs des revendications précédentes et comprenant un conteneur pour fragments (50) de dents pouvant être placé à l'intérieur de la chambre de réaction (22) capable de maintenir lesdits fragments de dent à son intérieur et perméable aux liquides pendant les réactions.
